# EUROPÄISCHE PATENTSCHRIFT

(11) **EP 2 513 873 B1**
(45) Veröffentlichungstag und Bekanntmachung des Hinweises auf die Patenterteilung: **07.10.2020**
(21) Anmeldenummer: 10792845.9
(22) Anmeldetag: 16.12.2010
(51) Int. Cl.: G07D 7/121, G07D 7/1205

(54) **SENSOR ZUR PRÜFUNG VON WERTDOKUMENTEN**
SENSOR FOR CHECKING VALUE DOCUMENTS
CAPTEUR POUR VÉRIFIER DES DOCUMENTS DE VALEUR

(30) Priorität: 18.12.2009 DE 102009058804
(43) Veröffentlichungstag der Anmeldung: 24.10.2012
(73) Patentinhaber: Giesecke+Devrient Currency Technology GmbH, 81677 München (DE)
(72) Erfinder: FRANKENBERGER, Jörg, 85570 Markt Schwaben (DE); DECKENBACH, Wolfgang, 83135 Schechen (DE); HENNERKES, Urs, 82256 Fürstenfeldbruck (DE); RAUSCHER, Wolfgang, 81677 München (DE)
(86) Internationale Anmeldenummer: PCT/EP2010/007703
(87) Internationale Veröffentlichungsnummer: WO 2011/072862

(56) Entgegenhaltungen:
- WO-A1-85/02928
- WO-A1-97/31340
- WO-A1-2005/109352
- DE-A1-102005 029 119
- DE-B3-102007 031 230
- DE-U1-202007 013 090
- JP-A- 2005 276 849
- JP-A- 2006 058 488
- US-A- 6 101 266

## Beschreibung

Die Erfindung betrifft einen Sensor zur Prüfung von Wertdokumenten und ein Verfahren zur Herstellung des Sensors aus einer Sensorplattform sowie eine Sensorfamilie mit mindestens zwei aus der gleichen Sensorplattform hergestellten Sensoren.

Zur Prüfung von Wertdokumenten werden üblicherweise Sensoren verwendet, mit denen die Art der Wertdokumente bestimmt wird und/oder mit denen die Wertdokumente auf Echtheit, und/ oder auf ihren Zustand geprüft werden. Derartige Sensoren werden zur Prüfung von Wertdokumenten wie z.B. Banknoten, Schecks, Ausweisen, Kreditkarten, Scheckkarten, Tickets, Gutscheinen und dergleichen verwendet. Die Prüfung der Wertdokumente erfolgt in einer Vorrichtung zur Wertdokumentbearbeitung, in der, je nach den zu prüfenden Wertdokumenteigenschaften, einer oder mehrere unterschiedliche Sensoren enthalten sind. Üblicherweise werden die Wertdokumente bei der Prüfung in einer oder mehreren Spuren abgetastet, wobei der Sensor und das Wertdokument relativ zueinander bewegt werden.

Die Wertdokumente werden häufig mit Hilfe optischer Sensoren geprüft, die das von den Wertdokumenten reflektierte Licht erfassen. Bisherige optische Sensoren sind auf die bei der Entwicklung des Sensors definierten spektralen Eigenschaften des Sensors festgelegt, wie z.B. die spektrale Auflösung und die Lage und Breite des durch den Sensor erfassbaren Spektralbereichs. Um einen derartigen optischen Sensor an andere Anforderungen, z.B. einen anderen Spektralbereich oder einer anderen spektralen Auflösung, anzupassen, sind grundlegende Veränderungen der optischen Komponenten des Sensors erforderlich. Bei optischen Sensoren, deren spektrale Selektion durch verschiedene Farbfilter erfolgt, die jeweils vor einem Detektor angeordnet sind, wäre z.B. ein Austausch der Farbfilter erforderlich, um den erfassbaren Spektralbereich zu verändern. Um bei einem solchen Sensor die spektrale Auflösung zu erhöhen, müssten zusätzliche Detektoren mit anderen Farbfiltern in den Sensor eingebaut werden. Dies ist jedoch üblicherweise aufgrund des begrenzten Platzes innerhalb des Sensorgehäuses kaum möglich.

Es ist auch bekannt, zur Beleuchtung eines Wertdokuments mehrere verschiedenfarbige Lichtquellen zu verwenden, deren Licht über einen gemeinsamen Lichtleiter auf das Wertdokument gerichtet wird. Aufgrund der Relativbewegung zwischen dem Sensor und dem daran vorbei transportierten Wertdokument ist jedoch ein Mindestabstand zwischen dem Lichtleiter und dem Sensor erforderlich. Da das Licht nach dem Austreten aus dem Lichtleiter divergiert, führt dieser Mindestabstand dazu, dass der beleuchtete Bereich auf dem Wertdokument relativ groß und die Beleuchtungsintensität entsprechend gering ist. Eine derartige Beleuchtung ist daher ungünstig, wenn die optischen Eigenschaften eines Wertdokuments in einem räumlich begrenzten Bereich erfasst werden sollen.

Die DE20 2007 013090 U1 offenbart ein Verfahren zur Herstellung eines Sensors und einen entsprechenden Sensor zur Prüfung von Wertdokumenten wobei eine Sensorplattform bereitgestellt wird, welche eine Lichtquellen-Aufnahme, auf der eine Vielzahl von Lichtquellen-Positionen vorgesehen sind, von denen jede zur Aufnahme einer Chip-förmigen Lichtquelle ausgebildet ist, ein Mikrolinsenarray, das eine Vielzahl von Mikrolinsen aufweist, wobei das Mikrolinsenarray und die Lichtquellen-Aufnahme so zueinander angeordnet werden können, dass jede der Lichtquellen-Positionen genau einer der Mikrolinsen zugeordnet wird, wobei die Mikrolinsen in dem Mikrolinsenarray in dem gleichen ein- oder zweidimensionalen Raster angeordnet sind wie die Lichtquellenpositionen auf der Lichtquellenaufnahme angeordnet sind und eine Detektionseinrichtung umfasst. Ferner wird eine Beleuchtungseinrichtung gebildet durch Ausstatten der Lichtquellen-Aufnahme mit chipförmigen Lichtquellen, wobei die Emissionsspektren von mindestens zwei der chipförmigen Lichtquellen voneinander verschieden sind, und durch Anordnen des Mikrolinsenarrays und der mit den chipförmigen Lichtquellen ausgestatteten Lichtquellen-Aufnahme so zueinander, dass jeder der chipförmigen Lichtquellen genau eine der Mikrolinsen des Mikrolinsenarrays zugeordnet wird.

Die Detektionseinrichtung wird dabei derart angeordnet, dass die Detektionseinrichtung Detektionslicht detektieren kann, welches, beim Betreiben des Sensors, von dem durch die Beleuchtungseinrichtung beleuchteten Wertdokument ausgeht.

Eine Aufgabe der vorliegenden Erfindung ist es daher, eine Sensorplattform für einen Sensor zur Prüfung von Wertdokumenten bereit zu stellen, aus der auf einfache Weise, für verschiedene spektrale Anforderungen, verschiedene Sensoren zur Erfassung der optischen Eigenschaften eines Wertdokuments in einem räumlich begrenzten Bereich hergestellt werden können.

Diese Aufgabe wird durch die Gegenstände der unabhängigen Ansprüche gelöst. In davon abhängigen Ansprüchen sind vorteilhafte Weiterbildungen und Ausgestaltungen der Erfindung angegeben.

Der Sensor ist zur Prüfung von Wertdokumenten ausgebildet und enthält eine Beleuchtungseinrichtung zum Beleuchten eines durch den Sensor zu prüfenden Wertdokuments, eine Abbildungsoptik und eine Detektionseinrichtung. Durch die Abbildungsoptik wird das von der Beleuchtungseinrichtung ausgesendete Licht auf das durch den Sensor zu prüfende Wertdokument abgebildet. Die Detektionseinrichtung ist zum Detektieren von Detektionslicht ausgebildet, das, beim Betreiben des Sensors, wenn das Wertdokument durch die Beleuchtungseinrichtung beleuchtet wird, von dem zu prüfenden Wertdokument ausgeht.

Die Beleuchtungseinrichtung weist eine Lichtquellen-Aufnahme auf, auf der eine Vielzahl von Lichtquellen-Positionen vorgesehen sind, von denen jede zur Aufnahme einer chipförmigen Lichtquelle ausgebildet ist. An mehreren der Lichtquellen-Positionen der Lichtquellen-Aufnahme ist jeweils genau eine Lichtquelle angeordnet. Die Lichtquellen-Positionen sind auf der Lichtquellen-Aufnahme nebeneinander angeordnet und werden z.B. durch eine Vielzahl individueller Vertiefungen definiert, durch welche jeweils genau eine Lichtquelle aufgenommen werden kann. Vorzugsweise sind die Vertiefungen so ausgebildet, dass jeweils eine chipförmige Lichtquelle darin einsetzbar ist. Die Lichtquellen-Positionen können aber auch durch Erhebungen und/ oder durch elektrische Kontaktflächen, die die Lichtquellen-Aufnahme aufweisen kann, definiert werden, die zur Aufnahme einer chipförmigen Lichtquelle ausgebildet sind. Um eine eineindeutige Zuordnung zwischen den Mikrolinsen und den an den Lichtquellen-Positionen anzuordnenden Lichtquellen zu erhalten, sind die Anordnung der Mikrolinsen in dem Mikrolinsenarray und die Anordnung der Lichtquellen-Positionen auf der Lichtquellenaufnahme gleich.

Mindestens zwei der auf der Lichtquellen-Aufnahme angeordneten Lichtquellen haben voneinander verschieden Emissionsspektren. Das heißt, mindestens eine der Lichtquellen weist ein Emissionsspektrum auf, dessen Maximum bei einer anderen Wellenlänge liegt als die Maxima der Emissionsspektren der anderen Lichtquellen, die auf der Lichtquellen-Aufnahme angeordnet sind. Beispielsweise ist jede Lichtquelle zur Emission einer Emissionslinie bei einer bestimmten Wellenlänge ausgebildet. Vorzugsweise weist die Beleuchtungseinrichtung eine Vielzahl verschiedener Lichtquellen auf, d.h. eine Vielzahl von Lichtquellen mit voneinander verschiedenen Emissionsspektren, deren Intensitätsmaxima bei verschiedenen Wellenlängen liegen. Insbesondere sind auf der Lichtquellen-Aufnahme eine Vielzahl von Lichtquellen angeordnet, deren Emissionsspektren im sichtbaren Spektralbereich liegen, und/oder eine Vielzahl von Lichtquellen, deren Emissionsspektren im infraroten Spektralbereich liegen und/oder deren Emissionsspektren im ultravioletten Spektralbereich liegen. Als Lichtquellen werden bevorzugt lichtemittierende Dioden eingesetzt, beispielsweise Leuchtdioden (LED), insbesondere Halbleiter-Leuchtdioden oder organische Leuchtdioden (OLED), und/oder Laserdioden, insbesondere vertikal-cavity surface emitting laser (VCSEL).

Außerdem weist die Beleuchtungseinrichtung des Sensors ein Mikrolinsenarray auf, das eine Vielzahl von Mikrolinsen enthält. Das Mikrolinsenarray und die Lichtquellen-Aufnahme sind derart zueinander angeordnet, dass jeder der auf der Lichtquellen-Aufnahme angeordneten Lichtquellen genau eine der Mikrolinsen zugeordnet ist. Beim Betreiben des Sensors wird so das Emissionslicht jeder der Lichtquellen durch genau eine Mikrolinse des Mikrolinsenarrays gesammelt. Jede dieser Mikrolinsen sammelt dabei nur das Emissionslicht genau einer der Lichtquellen auf. Durch die der jeweiligen Lichtquelle zugeordnete Mikrolinse wird das Emissionslicht jeder der Lichtquellen mit hoher Effizienz gesammelt.

Die Mikrolinsen sind in dem Mikrolinsenarray in dem gleichen ein- oder zweidimensionalen Raster angeordnet wie die Lichtquellen-Positionen auf der Lichtquellenaufnahme angeordnet sind. Insbesondere ist das Mikrolinsenarray als einstückiger Körper ausgebildet, der vorzugsweise Befestigungsmittel aufweist, die integraler Bestandteil des Mikrolinsenarrays, insbesondere des einstückigen Körpers sind. Die Lichtquellen-Aufnahme weist ein zu den Befestigungsmitteln des Mikrolinsenarrays passendes Gegenstück auf. Die Befestigungsmittel des Mikrolinsenarrays sind z.B. als Befestigungsstifte ausgebildet oder als Löcher zur Aufnahme von Befestigungsstiften, die auf der Lichtquellen-Aufnahme vorgesehen sind. Insbesondere sind alle Mikrolinsen des Mikrolinsenarrays zueinander koplanar angeordnet. Vorzugsweise sind alle Mikrolinsen des Mikrolinsenarrays gleich ausgebildet, um eine größtmögliche Variabilität in der Anordnung der Lichtquellen auf der Lichtquellen-Aufnahme zu gewährleisten. Insbesondere weisen dazu alle Mikrolinsen die gleiche Form und/ oder die gleiche Brennweite auf. Alternativ können auch einige Mikrolinsen des Mikrolinsenarrays eine von den übrigen Mikrolinsen abweichende Form und/oder Brennweite aufweisen. Dadurch könnte z.B. eine individuelle Anpassung der Mikrolinsen an die optischen Eigenschaften der Lichtquellen erreicht werden, denen sie zugeordnet sind und zu deren Lichtsammlung sie vorgesehen sind.

Durch Verwendung des Mikrolinsenarrays ergeben sich große Vorteile im Vergleich zu einer Beleuchtungseinrichtung, bei der für jede Lichtquelle eine Einzellinse verwendet wird. Denn in diesem Fall müsste für jede der Einzellinsen eine individuelle Halterung vorgesehen werden und bei der Befestigung der Einzellinsen die genaue Positionierung relativ zu der jeweiligen Lichtquelle sicher gestellt werden. Dabei kann es erforderlich sein, dass die genaue Position und/oder Orientierung der Einzellinsen nachträglich justiert werden muss. Demgegenüber reicht bei Verwendung eines Mikrolinsenarrays, das für jede Lichtquelle genau eine Mikrolinse aufweist, eine einzige genaue Positionierung aus. Diese Positionierung kann durch die Befestigungsmittel des Mikrolinsenarrays erfolgen, die mit den entsprechenden Gegenstücken der Lichtquellen-Aufnahme verbunden werden. Die Herstellung des Sensors kann daher viel einfacher und ohne Justage erfolgen. Im Gegensatz zur Realisierung einer entsprechenden Beleuchtung mit Einzellinsen, die einzeln gehaltert werden müssen und bei deren Anordnung immer Zwischenräume verbleiben, besteht bei dem Mikrolinsenarray außerdem zwischen den einzelnen Mikrolinsen kein oder nur ein minimaler Zwischenraum. Da das Mikrolinsenarray als einstückiger Körper ausgebildet ist, können die Mikrolinsen direkt ineinander übergehen. Durch das Mikrolinsenarray kann daher quasi eine flächendeckende Lichtsammlung erreicht werden. Durch das Mikrolinsenarray kann daher eine Beleuchtungseinrichtung gebildet werden, die eine hohe Lichtsammeleffizienz aufweist und sehr kompakt ist.

Der Sensor weist außerdem eine Abbildungsoptik auf, die dazu ausgebildet ist, das Emissionslicht jeder der Lichtquellen, nach Durchtritt durch die jeweilige Mikrolinse, auf ein durch den Sensor zu prüfendes Wertdokument abzubilden. Die Mikrolinsen und die Abbildungsoptik sind so zueinander angeordnet, dass das Emissionslicht jeder der Lichtquellen durch die jeweils zugeordnete Mikrolinse und die Abbildungsoptik auf ein Wertdokument abgebildet werden kann, das durch den Sensor erfasst werden soll. Die Abbildungsoptik weist bevorzugt ein oder mehrere refraktive und/oder diffraktive optische Elemente auf, die das Beleuchtungslicht auf das Wertdokument abbilden. Vorzugsweise ist die Abbildungsoptik als Abbildungslinse ausgebildet. Um das von der Beleuchtungseinrichtung ausgesendete Licht der verschiedenen Lichtquellen weitgehend auf denselben beleuchteten Bereich des Wertdokuments abzubilden, wird die Abbildungsoptik vorzugsweise so angeordnet, dass der beleuchtete Bereich des Wertdokuments genau oder näherungsweise im Brennpunkt der Abbildungsoptik liegt. Dadurch kann erreicht werden, dass trotz der Beleuchtung des Wertdokuments mit verschiedenen Lichtquellen, stets derselbe Bereich des zu prüfenden Wertdokuments beleuchtet und durch die Detektionseinrichtung detektiert werden kann.

Die Abbildungsoptik ist auf der dem Wertdokument zugewandten Seite des Mikrolinsenarrays angeordnet. Die Abbildungsoptik ist dazu ausgebildet, das Emissionslicht jeder der Lichtquellen, nach Durchtritt durch die jeweilige Mikrolinse, auf ein durch den Sensor zu prüfendes Wertdokument abzubilden. Das von der Beleuchtungseinrichtung ausgesendete Licht wird durch die Abbildungsoptik über einen definierten Strahlengang auf den beleuchteten Bereich der Messebene bzw. des Wertdokuments abgebildet. Dadurch dass eine Abbildung des Beleuchtungslichts auf das Wertdokument erfolgt, ist der beleuchtete Bereich des Wertdokuments klar definiert und räumlich begrenzt. Dies stellt einen Vorteil gegenüber einer direkten Beleuchtung des Wertdokuments durch die Lichtquellen (ohne dazwischen liegende Optik) dar und gegenüber einer einfachen Lichtleiteroptik (ohne Abbildungsoptik), durch die das Licht nicht abgebildet wird, sondern ohne definierten Strahlengang vom Lichtleiter auf das Wertdokument gebracht wird.

Für den Sensor ist eine Steuerungseinrichtung vorgesehen, die dazu eingerichtet ist, die Lichtquellen der Beleuchtungseinrichtung nacheinander ein- und wieder auszuschalten, um das Wertdokument nacheinander mit verschiedenen Emissionsspektren zu beleuchten. Die Steuerungseinrichtung kann als Bestandteil des Sensors ausgebildet sein, sie kann aber auch als externe Steuerungseinrichtung, z.B. als Bestandteil einer Vorrichtung zur Wertdokumentbearbeitung, ausgebildet sein, in die der Sensor eingebaut wird. Die Steuereinrichtung ist dazu eingerichtet, die Beleuchtungseinrichtung des Sensors, insbesondere die Lichtquellen, und die Detektionseinrichtung des Sensors anzusteuern. Beim Betreiben des Sensors schaltet die Steuerungseinrichtung die Lichtquellen nacheinander ein und wieder aus, beispielsweise so, dass zu jedem Zeitpunkt genau eine der Lichtquellen eingeschaltet ist. Zu einem oder mehreren der Zeitpunkte können aber auch gleichzeitig mehrere der Lichtquellen eingeschaltet sein, z.B. mehrere Lichtquellen mit gleichem Emissionsspektrum. Das Wertdokument wird auf diese Weise nacheinander mit den verschiedenen Emissionsspektren der verschiedenen Lichtquellen beleuchtet. Außerdem veranlasst die Steuereinrichtung, dass die Detektionseinrichtung während der eingeschalteten Phase der Lichtquellen jeweils einen Messwert erfasst, der der von dem Wertdokument ausgehenden Lichtintensität entspricht. Da die Detektionseinrichtung synchron zur Beleuchtung durch die Lichtquellen jeweils einen Messwert aufnimmt, wird so für diejenigen Wellenlängen, die durch das Emissionslicht der Lichtquellen vorgeben sind, die von dem Wertdokument ausgehende Lichtintensität detektiert. Bevorzugt weist der Sensor eine Vielzahl verschiedener Lichtquellen auf, die, beim Betreiben des Sensors, nacheinander ein- und ausgeschaltet werden, um die spektrale Intensitätsverteilung des von dem Wertdokument ausgehenden Lichts zu erfassen.

Die Detektionseinrichtung weist vorzugsweise eine spektrale Empfindlichkeit auf, die spektral so breitbandig ist, dass durch die Detektionseinrichtung das Emissionslicht jeder der Lichtquellen der Beleuchtungseinrichtung detektierbar ist. Insbesondere ist durch die Detektionseinrichtung zumindest sichtbares Licht und/ oder infrarotes und/oder ultraviolettes Licht detektierbar. Die von der Detektionseinrichtung aufgenommenen Messwerte werden anschließend durch eine Auswerteeinrichtung ausgewertet, die Bestandteil des Sensors sein kann oder auch durch eine externe Auswerteeinrichtung gebildet wird. Vorzugsweise erfolgt bereits durch den Sensor, insbesondere durch eine interne Auswerteeinrichtung des Sensors, zumindest eine Vorverarbeitung der Messwerte. Die weitere Auswertung kann ebenfalls durch die interne Auswerteeinrichtung oder alternativ durch eine zentrale Auswerteeinrichtung der Vorrichtung erfolgen, in die der Sensor eingebaut ist. Zur Detektion des Remissionslichts kann der Sensor auch mehrere gleiche Detektionseinrichtungen enthalten, z.B. um das Remissionslicht über einen größeren Winkelbereich zu erfassen. Der Sensor kann auch mehrere verschiedene Detektionseinrichtungen aufweisen, z.B. um den mit dem Sensor erfassbaren Spektralbereich zu erweitern. Die verschiedenen Detektionseinrichtungen können dabei nebeneinander angeordnet sein oder hintereinander, z.B. in Form einer Sandwich-Struktur.

Durch die Erfindung wird außerdem ein Verfahren zur Herstellung eines Sensors zur Prüfung von Wertdokumenten aufgezeigt, durch das der herzustellende Sensor für verschiedene spektrale Anforderungen mit geringem Aufwand maßgeschneidert werden kann.

Der Sensor wird aus einer Sensorplattform hergestellt. Die Sensorplattform umfasst zumindest die oben genannte Lichtquellen-Aufnahme mit der Vielzahl von Lichtquellen-Positionen, das Mikrolinsenarray mit der Vielzahl von Mikrolinsen, die Abbildungsoptik und die Detektionseinrichtung. Um die Beleuchtungseinrichtung zu bilden, wird die Lichtquellen-Aufnahme mit Lichtquellen ausgestattet, wobei an mehreren der Lichtquellen-Positionen jeweils genau eine Lichtquelle angeordnet wird. Mindestens zwei der Lichtquellen, vorzugsweise eine Vielzahl der Lichtquellen, weisen voneinander verschiedene Emissionsspektren auf. Zum Bilden der Beleuchtungseinrichtung werden anschließend das Mikrolinsenarray und die Lichtquellen-Aufnahme so zueinander angeordnet, dass jeder der auf der Lichtquellen-Aufnahme angeordneten Lichtquellen genau eine der Mikrolinsen zugeordnet wird und so, dass, beim Betreiben des Sensors, das Emissionslicht jeder der Lichtquellen durch genau eine Mikrolinse des Mikrolinsenarrays gesammelt wird. Bevorzugt werden dabei das Mikrolinsenarray und die Lichtquellen-Aufnahme aneinander befestigt. Zur Herstellung des Sensors wird außerdem die Abbildungsoptik derart relativ zu der Beleuchtungseinrichtung angeordnet, dass das Emissionslicht jeder der an den Lichtquellen-Positionen aufgenommenen Lichtquellen, nach Durchtritt durch die jeweilige Mikrolinse, durch die Abbildungsoptik auf das zu prüfende Wertdokument abgebildet werden kann. Außerdem werden zur Herstellung des Sensors die Beleuchtungseinrichtung, die Abbildungsoptik und die Detektionseinrichtung derart zueinander angeordnet, dass die Detektionseinrichtung Detektionslicht detektieren kann, welches, beim Betreiben des Sensors, von dem durch die Beleuchtungseinrichtung beleuchteten Wertdokument ausgeht.

Beim Konfigurieren des Sensors werden Beleuchtungssequenzen festgelegt, wobei insbesondere festgelegt wird, welche Lichtquellen zur Beleuchtung des Wertdokuments ein- und ausgeschaltet werden. Die Steuereinrichtung wird dabei so konfiguriert, dass die Lichtquellen der Beleuchtungseinrichtung, beim Betreiben des Sensors, nacheinander ein- und ausgeschaltet werden, so dass das Wertdokument nacheinander mit verschiedenen Emissionsspektren beleuchtet werden kann. Die für den Sensor vorgesehene Steuerungseinrichtung kann bereits bei der Herstellung des Sensors konfiguriert werden. Es kann jedoch vorgesehen sein, dass die Konfiguration der Steuereinrichtung, erst nach der Fertigstellung des Sensors durchgeführt wird. Ferner kann vorgesehen sein, dass die Konfiguration der Steuereinrichtung auch nach Inbetriebnahme des Sensors veränderbar ist. Ein derartiges Umkonfigurieren nach der Inbetriebnahme kann z.B. durch den Hersteller des Sensors oder durch eine Bedienperson der Vorrichtung, in der der Sensor eingebaut ist, durchgeführt werden. Beim Umkonfigurieren kann es auch notwendig sein, die Ansteuerung der Detektionseinrichtung an die Ansteuerung der Beleuchtung anzupassen, z.B. wenn die Anzahl der zur Messung ein- und ausgeschalteten Lichtquellen verändert wird. Beim Umkonfigurieren ist auch die Auswerteeinrichtung, die zur Auswertung der aufgenommenen Messwerte verwendet wird, an die veränderte Konfiguration der Steuereinrichtung anzupassen, z.B. wenn andere Lichtquellen zur Messung verwendet werden.

Der Sensor ist nicht zur vollflächigen Prüfung des Wertdokuments, sondern zur Prüfung des Wertdokuments in einer oder in mehreren Spuren auf dem Wertdokument ausgebildet. Im Fall der Prüfung in mehreren Spuren sind zwischen den Spuren jeweils Wertdokumentbereiche angeordnet, die durch den Sensor nicht geprüft werden. Die zur Prüfung des Wertdokuments beleuchteten Bereiche bilden Spuren, die parallel zueinander und entlang der Transportrichtung des Wertdokuments verlaufen. Die Spuren sind auf dem Wertdokument diskret verteilt. Für jede der Spuren ist zumindest eine Beleuchtungseinrichtung, eine Abbildungsoptik und eine Detektionseinrichtung gemäß der obigen Beschreibung vorgesehen. Die Beleuchtungssequenzen folgen vorzugweise so schnell aufeinander, dass das Wertdokument entlang jeder der Spuren quasi kontinuierlich geprüft wird.

Optional kann vor der Detektionseinrichtung eine Detektionsoptik angeordnet sein, durch die das von dem Wertdokument ausgehende Detektionslicht gesammelt und auf den lichtempfindlichen Bereich gerichtet wird. Die Detektionsoptik kann z.B. durch refraktive oder diffraktive optische Elemente oder durch Spiegel realisiert sein. Vorzugsweise weist der Sensor außerdem ein Gehäuse auf, in dem die Beleuchtungseinrichtung, die Abbildungsoptik und die Detektionseinrichtung, optional auch die Steuereinrichtung und Detektionsoptik, angeordnet sind.

Der Sensor wird aus einer Sensorplattform hergestellt, die durch die Lichtquellen-Aufnahme, das Mikrolinsenarray, die Abbildungsoptik und die Detektionseinrichtung gebildet wird. Optional gehört zur Sensorplattform auch die Steuerungseinrichtung, die innerhalb oder außerhalb des Gehäuses angeordnet sein kann. Diese ist jedoch nicht notwendigerweise ein Bestandteil des Sensors. Optional können zur Sensorplattform auch das Gehäuse und/oder die Detektionsoptik gehören. Der Sensor kann aber auch ohne Gehäuse und/oder ohne Detektionsoptik realisiert werden. Aus der Sensorplattform können, je nach Auswahl der Lichtquellen, mit der die Lichtquellen-Aufnahme ausgestattet wird, verschiedene Sensoren hergestellt werden. Je nach den spektralen Anforderungen, die an den herzustellenden Sensor gestellt werden, kann aus der gleichen Sensorplattform ein Sensor erster Art hergestellt werden, der zur Prüfung von Wertdokumenten durch Beleuchtung mit mehreren ersten Emissionsspektren ausgebildet ist, oder ein Sensor zweiter Art, der zur Prüfung von Wertdokumenten durch Beleuchtung mit mehreren zweiten Emissionsspektren ausgebildet ist, von denen sich mindestens eines von den ersten Emissionsspektren unterscheidet.

Die Erfindung betrifft außerdem eine Sensorfamilie, die durch mehrere verschiedene, aber aus der gleichen Sensorplattform herstellbare Sensoren zur Prüfung von Wertdokumenten gebildet wird. Die Sensorfamilie umfasst mindestens einen Sensor erster Art, der zur Prüfung von Wertdokumenten durch Beleuchtung mit einer Vielzahl erster Emissionsspektren ausgebildet ist, und mindestens eine Sensor zweiter Art, der zur Prüfung von Wertdokumenten durch Beleuchtung mit einer Vielzahl zweiter Emissionsspektren ausgebildet ist, von denen sich mindestens eines von den ersten Emissionsspektren unterscheidet. Der mindestens eine Sensor erster Art und der mindestens eine Sensor zweiter Art weisen zum Beispiel das gleiche Mikrolinsenarray und/oder die gleiche Lichtquellen-Aufnahme und/oder die gleiche Abbildungsoptik und/oder die gleiche Detektionseinrichtung auf. Vorzugsweise sind der mindestens eine Sensor erster Art und der mindestens eine Sensor zweiter Art aus der gleichen Sensorplattform herstellbar, wobei diese Sensorplattform durch die Lichtquellen-Aufnahme, das Mikrolinsenarray, die Abbildungsoptik und die Detektionseinrichtung gebildet wird. Die Sensoren erster Art und die Sensoren zweiter Art weisen in diesem Fall die gleiche Lichtquellen-Aufnahme auf, auf der jeweils eine Vielzahl von Lichtquellen-Positionen vorgesehen sind, von denen jede zur Aufnahme einer Lichtquelle ausgebildet ist, und/oder das gleiche Mikrolinsenarray, das jeweils eine Vielzahl von Mikrolinsen aufweist, wobei das Mikrolinsenarray und die Lichtquellen-Aufnahme so zueinander angeordnet werden können, dass jede der Lichtquellen-Positionen genau einer der Mikrolinsen zugeordnet wird, und die gleiche Abbildungsoptik und die gleiche Detektionseinrichtung.

In einem Ausführungsbeispiel ist auch die Auswahl der Lichtquellen, mit denen die Sensoren erster Art und die Sensoren zweiter Art ausgestattet sind, die gleiche. Die Sensoren erster Art und die Sensoren zweiter Art sind jedoch verschieden konfiguriert, so dass, beim Betreiben der Sensoren zweiter Art, eine oder mehrere andere Lichtquellen ein- und ausgeschaltet werden als beim Betreiben der Sensoren erster Art. Die Sensoren zweiter Art sind so konfiguriert, dass sie zur Prüfung der Wertdokumente eine andere Teilmenge der Lichtquellen verwenden als die Sensoren erster Art. Zum Beispiel prüfen die Sensoren zweiter Art die Wertdokumente mit Licht einer oder mehrerer Wellenlängen, die die Sensoren erster Art zur Prüfung der Wertdokumente nicht verwenden.

In einem anderen Ausführungsbeispiel sind die Anzahl und Auswahl der Lichtquellen, mit denen die Sensoren erster Art und die Sensoren zweiter Art ausgestattet sind, unterschiedlich. Die Sensoren erster Art sind z.B. mit einer ersten Auswahl an Lichtquellen ausgestattet sein und die Sensoren zweiter Art mit einer zweiten Auswahl an Lichtquellen, die sich von der ersten Auswahl unterscheidet. Insbesondere weisen eine oder mehrere Lichtquellen der zweiten Auswahl jeweils ein Emissionsspektrum auf, dessen spektrale Lage verschieden ist im Vergleich zur spektralen Lage aller Emissionsspektren der Lichtquellen der ersten Auswahl.

Neben den Sensoren erster Art und zweiter Art kann die Sensorfamilie auch einen oder mehrere Sensoren einer oder mehrerer weiterer Arten aufweisen, die aus der gleichen Sensorplattform hergestellt sind und ebenfalls zur Prüfung von Wertdokumenten ausgebildet sind. Analog zu den Sensoren zweiter Art können auch die zu der Sensorfamilie gehörigen Sensoren der weiteren Arten entweder mit der gleichen Auswahl an Lichtquellen ausgestattet sein und anders konfiguriert sein oder mit einer anderen Auswahl an Lichtquellen ausgestattet sein.

Nachfolgend wird die Erfindung beispielhaft anhand der folgenden Figuren erläutert. Es zeigen:
- Fig. 1a: Eine Lichtquellen-Aufnahme mit einer Vielzahl von Lichtquellen-Positionen und ein zu der Lichtquellen-Aufnahme zugehöriges Mikrolinsenarray,
- Fig. 1b: die Lichtquellen-Aufnahme aus Figur 1a mit darauf angeordneten Lichtquellen und das zugehörige Mikrolinsenarray,
- Fig. 1c: einen Schnitt durch eine aus der Lichtquellen-Aufnahme, den Lichtquellen und dem zugehörigen Mikrolinsenarray der Figur 1b gebildete Beleuchtungseinrichtung,
- Fig. 2a: ein aus einer Sensorplattform hergestellter Sensor, der die Beleuchtungseinrichtung aus Figur 1c zur Beleuchtung eines Wertdokuments verwendet,
- Fig. 2b: eine Sensorplattform, aus der verschiedene Sensoren zur Prüfung von Wertdokumenten hergestellt werden können,
- Fig. 3a-c: eine Sensorfamilie gemäß einem ersten Ausführungsbeispiel, bei dem die Sensoren erster Art und die Sensoren zweiter Art unterschiedliche Teilmengen der gleichen Auswahl an Lichtquellen verwenden,
- Fig. 4a-b: eine Sensorfamilie gemäß einem zweiten Ausführungsbeispiel, bei dem die Sensoren erster Art und die Sensoren zweiter Art mit einer unterschiedlichen Auswahl an Lichtquellen ausgestattet sind.

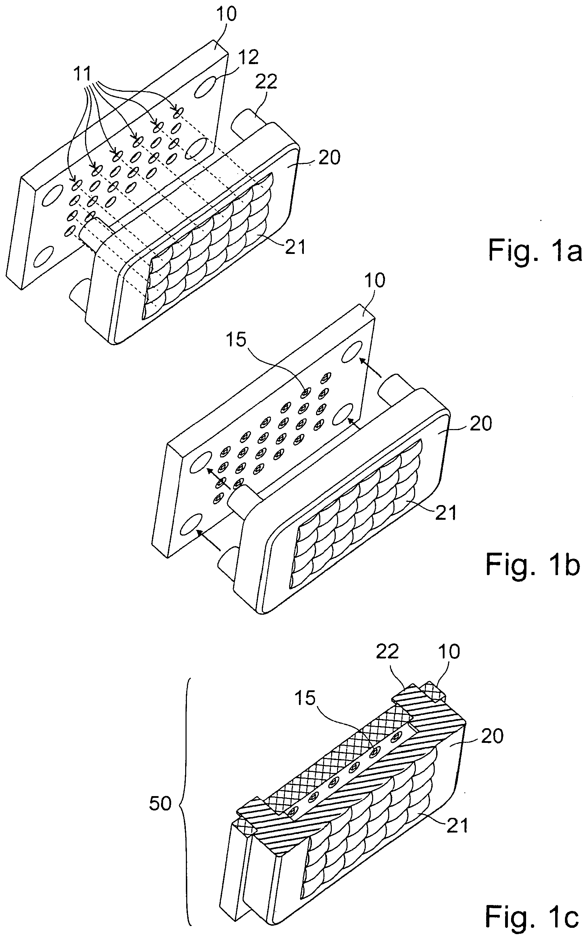

Figur 1a zeigt eine Lichtquellen-Aufnahme 10, auf der eine Vielzahl von Lichtquellen-Positionen 11 vorgesehen sind, von denen jede zur Aufnahme einer Lichtquelle 15 ausgebildet ist. Die Lichtquellen-Aufnahme 10 ist z.B. als Leiterplatte ausgebildet und weist eine zum Betreiben der Lichtquellen 15 benötigte elektrische Verdrahtungsstruktur auf (nicht gezeigt), die eine selektive Ansteuerung jeder einzelnen Lichtquelle erlaubt. Die Lichtquellen-Positionen 11 sind in diesem Beispiel durch Vertiefungen in der Lichtquellen-Aufnahme gebildet, in denen jeweils eine Lichtquelle 15 befestigt werden kann. Außerdem zeigt Figur 1a ein zu der Lichtquellen-Aufnahme 10 gehöriges Mikrolinsenarray 20, das eine Vielzahl von Mikrolinsen 21 aufweist. Die Lichtquellen-Aufnahme 10 und das Mikrolinsenarray sind so aufeinander abgestimmt, dass jeder der Lichtquellen-Positionen 11 genau eine der Mikrolinsen 21 zugeordnet ist. Zu diesem Zweck sind die Mikrolinsen 21 innerhalb des Mikrolinsenarrays in demselben zweidimensionalen Raster angeordnet wie die Lichtquellen-Positionen auf der Lichtquellen-Aufnahme 10 angeordnet sind. Das Mikrolinsenarray 20 ist als einstückiger Körper ausgebildet und wird beispielsweise durch einen Glaskörper oder durch einen transparenten Kunststoffkörper gebildet. Der Durchmesser der einzelnen Mikrolinsen liegt z.B. im µm-Bereich oder im mm-Bereich.

Zur Bildung einer Beleuchtungseinrichtung 50 werden einige oder alle Lichtquellen-Positionen 11 mit jeweils einer Lichtquelle 15 versehen, vgl. Figur 1b. Als Lichtquellen 15 werden z.B. LEDs und/oder OLEDs und/ oder VCSELs eingesetzt. Die Lichtquellen 15 weisen eine Vielzahl verschiedener Emissionsspektren auf. Beispielsweise weist jede der Lichtquellen 15 ein anderes Emissionsspektrum auf als die übrigen Lichtquellen 15. Alternativ dazu können aber auch mehrere gleiche Lichtquellen 15 verwendet werden, z.B. um auch in einem Spektralbereich mit lichtschwachen Lichtquellen eine ausreichende Beleuchtungsintensität zu erhalten.

Zur Befestigung des Mikrolinsenarrays 20 ist der Körper des Mikrolinsenarrays 20 mit Befestigungsstiften 22 ausgestattet, die in dazu passenden Löchern 12 in der Lichtquellen-Aufnahme 10 eingesteckt werden. Nach dem Einstecken der Befestigungsstifte 22 werden das Mikrolinsenarray 20 und die Lichtquellen-Aufnahme 10 aneinander fixiert, z.B. durch eine Formschlussverbindung oder durch Klebung. Die Lichtquellen-Aufnahme 11 mit den darauf angeordneten Lichtquellen 15 und das auf der Lichtquellen-Aufnahme 11 befestigte Mikrolinsenarray 20 bilden eine Beleuchtungseinrichtung 50, vgl. Figur 1c. Das von den einzelnen Lichtquellen 15 emittierte Licht wird durch die vor der jeweiligen Lichtquelle 15 angeordnete Mikrolinse 21 gesammelt. Die Lichtquellen-Aufnahme 10 ist so ausgeführt, dass die Lichtquellen-Positionen 11 relativ zu den Positionen der Löcher 12 der Lichtquellen-Aufnahme 10 mit möglichst geringen Toleranzen behaftet sind. Da die Befestigungsstifte 22 als integrale Bestandteile des Körpers des Mikrolinsenarrays 20 ausgebildet sind, ist deren Position relativ zu den Mikrolinsen 21 sehr genau definiert. Auf diese Weise wird durch die Befestigung des Mikrolinsenarrays 20 mittels der Befestigungsstifte 22 automatisch die optimale Position des Mikrolinsenarray 20 relativ zu den Lichtquellen 15 erreicht. Bei der Herstellung des Sensors 100 ist daher keine Justage der Beleuchtungseinrichtung 50 erforderlich.

Der Sensor zur Prüfung von Wertdokumenten wird im Folgenden am Beispiel eines Remissionssensors erläutert. Der erfindungsgemäße Sensor kann jedoch auch als Transmissionssensor ausgebildet sein. Dazu wird die Detektionseinrichtung gegenüber liegend zur Beleuchtungseinrichtung angeordnet werden, so dass das durch das Wertdokument transmittierte Beleuchtungslicht detektiert wird.

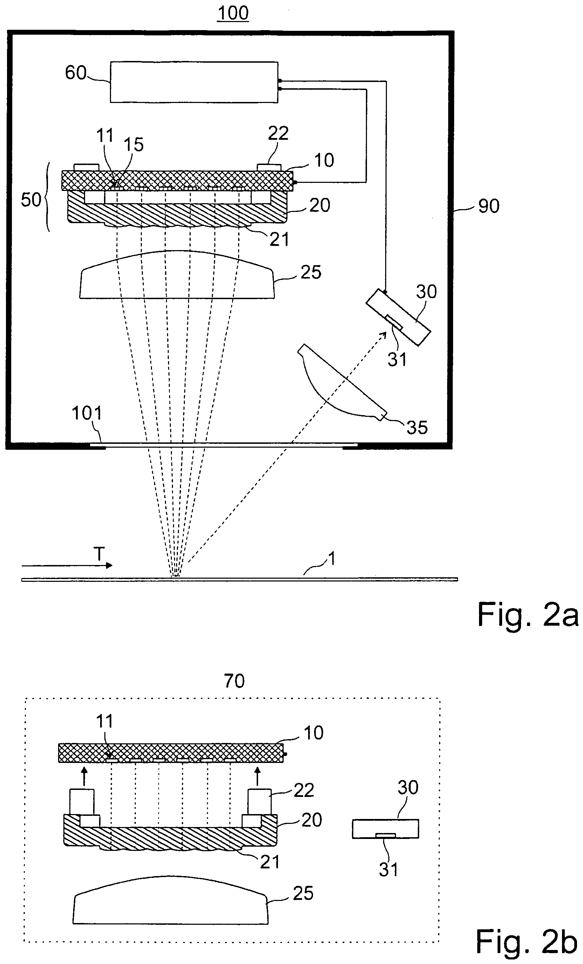

Die Beleuchtungseinrichtung 50 wird in einen Sensor 100 eingebaut, der zur Prüfung von Wertdokumenten ausgebildet ist, vgl. Figur 2a. Das von der Beleuchtungseinrichtung 50 emittierte Licht wird durch eine Abbildungslinse 25 auf das Wertdokument 1 abgebildet. Dabei wird das Wertdokument 1 über einen bestimmten Bereich mit homogener Lichtintensität beleuchtet. Um das von der Beleuchtungseinrichtung 50 emittierte Licht auf das Wertdokument 1 zu richten, können als Abbildungsoptik, alternativ zu der Abbildungslinse 25, aber auch andere optische Komponenten verwendet werden, z.B. Linsensysteme, diffraktive optische Komponenten, z.B. eine Fresnellinse, oder abbildende Spiegel. Von dem Wertdokument 1 werden, in Abhängigkeit der optischen Eigenschaften des Wertdokuments 1, Anteile des Beleuchtungslichts remittiert. Das von dem Wertdokument 1 remittierte Licht wird mit Hilfe einer Detektionseinrichtung 30 detektiert, die einen lichtempfindlichen Bereich 31 aufweist. Die Detektionseinrichtung 30 kann z.B. durch eine Photodiode oder einen Phototransistor gebildet sein. Optional kann vor der Detektionseinrichtung 30 eine Detektionsoptik 35 angeordnet sein, durch die das von dem Wertdokument 1 remittierte Licht gesammelt und auf den lichtempfindlichen Bereich 31 gerichtet wird. Im gezeigten Beispiel wird das Beleuchtungslicht senkrecht auf das Wertdokument 1 abgebildet und die Detektionseinrichtung 30 erfasst das unter schrägem Winkel remittierte Licht. Alternativ kann auch die Beleuchtung unter schrägem Winkel erfolgen und die Detektionseinrichtung das in senkrechter Richtung oder das in schräger Richtung remittierte Licht erfassen.

Der Sensor 100 weist ein Gehäuse 90 auf, an dessen Unterseite ein transparentes Fenster 101 angeordnet ist. Das von der Beleuchtungseinrichtung 50 emittierte Licht wird durch das Fenster 101 auf ein zu prüfendes Wertdokument 1 abgebildet, welches an dem Sensor 100 entlang einer Transportrichtung T vorbeitransportiert wird. Die Beleuchtungseinrichtung 50, insbesondere die Lichtquellen 15, und die Detektionseinrichtung 30 werden von einer Steuereinrichtung 60 angesteuert, die in diesem Beispiel innerhalb des Gehäuses 90 angeordnet ist. Die Steuerungseinrichtung 60 schaltet die Lichtquellen 15 nacheinander ein und wieder aus, zum Beispiel so, dass zu jedem Zeitpunkt jeweils genau eine Lichtquelle 15 eingeschaltet ist. Während der eingeschalteten Phase der Lichtquellen erfasst die Detektionseinrichtung 30 jeweils einen Messwert, der der von dem Wertdokument 1 remittierten Lichtintensität entspricht. Das Wertdokument 1 wird nacheinander mit den verschiedenen Emissionsspektren der verschiedenen Lichtquellen 15 beleuchtet. Da die Detektionseinrichtung 30 synchron zur Beleuchtung durch die Lichtquellen 15 jeweils einen Messwert aufnimmt, wird so in jedem Spektralbereich, den die Lichtquellen 15 vorgeben, die von dem Wertdokument 1 remittierte Lichtintensität gemessen.

Die Steuereinrichtung 60 steuert die Lichtquellen 15 so an, so dass sich die Beleuchtungssequenz, mit der die Lichtquellen 15 ein- und ausgeschaltet werden periodisch wiederholt. Beispielsweise kann die Steuereinrichtung 60 so programmiert sein, dass während jeder Beleuchtungssequenz jede Lichtquelle 15 der Beleuchtungseinrichtung 50 genau einmal ein- und ausgeschaltet wird. Alternativ kann eine Lichtquelle 15 auch mehrmals pro Beleuchtungssequenz angesteuert werden, z.B. um die geringe Intensität einer intensitätsschwachen Lichtquelle 15 durch mehrfache Messung zu kompensieren. Eine Beleuchtungssequenz kann entweder die Ansteuerung aller in der Beleuchtungseinrichtung 50 vorhandenen Lichtquellen 15 beinhalten oder nur einer Teilmenge der vorhandenen Lichtquellen 15. Nach einer Beleuchtungssequenz, d.h. nachdem unter Beleuchtung mit jedem Emissionsspektrum, das für die Messung vorgesehen ist, ein Messwert aufgenommen wurde, startet die nächste Beleuchtungssequenz, in der erneut unter Beleuchtung mit jedem Emissionsspektrum, das für die Messung vorgesehen ist, ein Messwert aufgenommen wird, usw. Die Dauer der Beleuchtungssequenz ist derart auf die Transportgeschwindigkeit des Wertdokuments 1 abgestimmt, dass die verschiedenen Messwerte einer Beleuchtungssequenz zumindest näherungsweise von demselben Detektionsbereich auf dem Wertdokument 1 stammen. Die Strecke, die das Wertdokument 1 vom Beginn bis zum Ende derselben Beleuchtungssequenz zurücklegt, ist also viel kürzer als die Länge des Detektionsbereichs. Die während einer Beleuchtungssequenz erhaltenen Messwerte können z.B. eine spektrale Abhängigkeit der Remission des jeweiligen Detektionsbereichs liefern.

Der Sensor 100 wird aus einer Sensorplattform 70 hergestellt, die aus folgenden Elementen besteht, vgl. Figur 2b: die Lichtquellen-Aufnahme 10, das Mikrolinsenarray 20, die Abbildungsoptik 25 und die Detektionseinrichtung 30. Optional gehören zur Sensorplattform 70 die Steuerungseinrichtung 60 und/oder das Gehäuse 90 und/oder die Detektionsoptik 35. Der erfindungsgemäße Sensor kann aber auch ohne diese Elemente realisiert werden. Aus der Sensorplattform 70 können, je nach Auswahl der Lichtquellen 15, mit der die Lichtquellen-Aufnahme 10 ausgestattet wird, verschiedene Sensoren hergestellt werden. Je nach Anforderungen, die an den herzustellenden Sensor gestellt werden, kann aus derselben Sensorplattform 70 zum Beispiel entweder ein Sensor erster Art 100A oder ein Sensor zweiter Art 100B hergestellt werden.

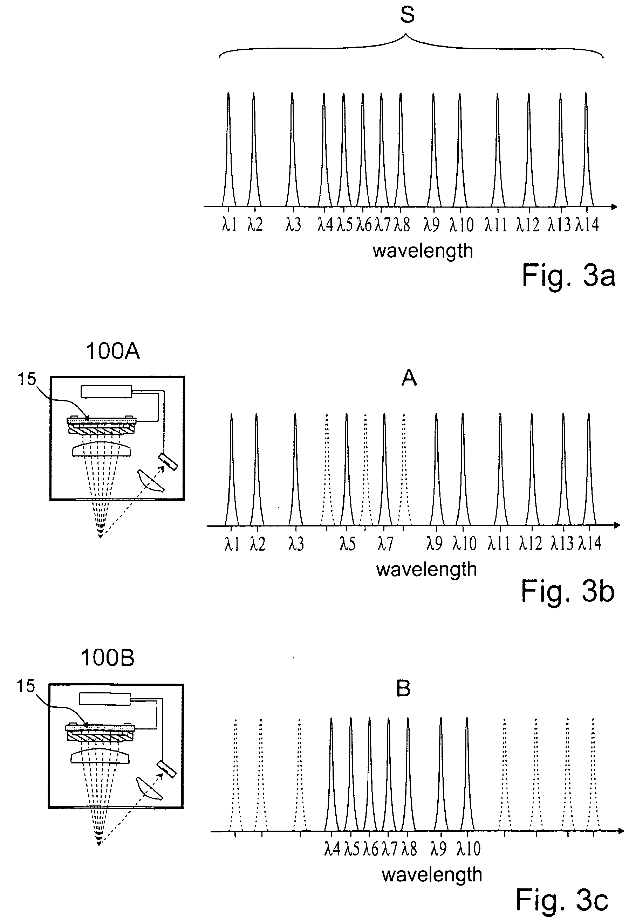

In einem ersten Ausführungsbeispiel sind die Sensoren erster Art 100A und die Sensoren zweiter Art 100B mit der gleichen Auswahl S an Lichtquellen 15 ausgestattet, vgl. Figur 3a. Die Auswahl S an Lichtquellen 15, mit der die Sensorplattform 70 bei der Herstellung der Sensoren 100A und 100B ausgestattet werden, besteht in dem gezeigten Beispiel jeweils aus 14 verschiedenen Lichtquellen 15. Die spektrale Lage der verschiedenen Emissionsmaxima dieser Lichtquellen 15 ist in Figur 3a mit λ1 bis λ14 bezeichnet. Die Emissionsspektren der Lichtquellen 15 erstrecken sich z.B. vom ultravioletten über den sichtbaren bis in den nahinfraroten Spektralbereich. Von der Auswahl S verwenden die Sensoren erster Art 100A und die Sensoren zweiter Art 100B jedoch verschiedene Teilmengen an Lichtquellen 15. Zum Beispiel verwendet der Sensor erster Art 100A die Teilmenge A, die aus den Lichtquellen mit den Wellenlängen λ1-λ3, λ5, λ7 und λ9-λ14 besteht, vgl. Figur 3b. Der Sensor zweiter Art 100B verwendet die Teilmenge B, die aus den Lichtquellen mit den Wellenlängen λ4-λ10 besteht, vgl. Figur 3c. Die Steuerungseinrichtung 60 des Sensors 100A ist so konfiguriert, dass während einer Beleuchtungssequenz nacheinander die Lichtquellen der Wellenlängen λ1-λ3, λ5, λ7 und λ9-λ14 an- und ausgeschaltet werden. Dagegen ist die Steuerungseinrichtung 60 des Sensors zweiter Art 100B so konfiguriert, dass während einer Beleuchtungssequenz des Sensors zweiter Art 100B nacheinander die Lichtquellen der Wellenlängen λ4-λ10 ein- und ausgeschaltet werden.

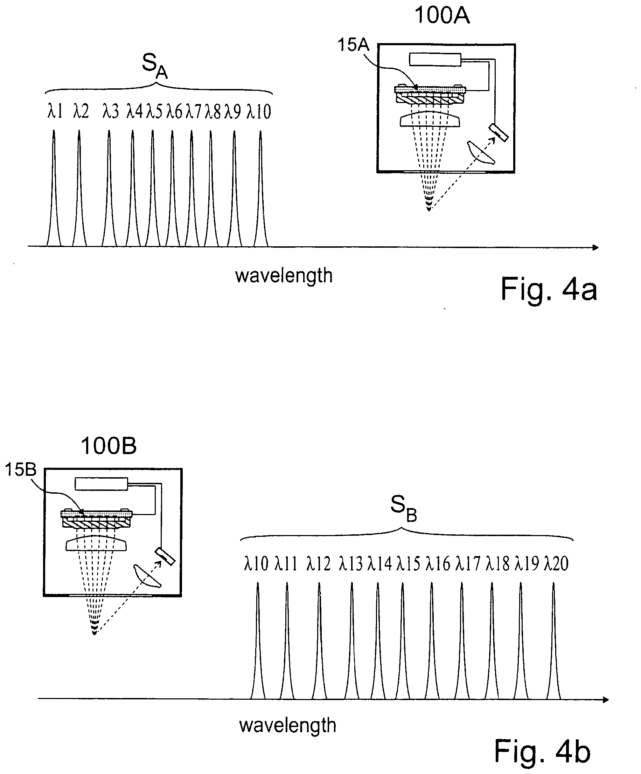

In einem zweiten Ausführungsbeispiel weisen die Sensoren zweiter Art eine andere Auswahl an Lichtquellen auf als die Sensoren erster Art 100A. Zur Herstellung der Sensoren 100B wird die Sensorplattform 70 also mit einer anderen Auswahl an Lichtquellen 15 ausgestattet wie zur Herstellung der Sensoren 100A. Beispielsweise sind die Sensoren erster Art 100A mit einer ersten Auswahl S_{A} an Lichtquellen 15A ausgestattet und die Sensoren zweiter Art 100B mit einer zweiten Auswahl S_{B} an Lichtquellen 15B, vgl. Figuren 4a-b. Die erste Auswahl S_{A} besteht in diesem Beispiel aus 10 verschiedenen Lichtquellen 15A, deren Wellenlängen mit λ1-λ10 bezeichnet sind. Die zweite Auswahl S_{B} besteht in diesem Beispiel aus 11 verschiedenen Lichtquellen 15B, deren Wellenlängen mit λ10-λ20 bezeichnet sind. Die Lichtquellen 15B können dabei teilweise mit den Lichtquellen 15A gleich sein.

## Patentansprüche

1. Verfahren zur Herstellung eines Sensors (100), der zur Prüfung von Wertdokumenten ausgebildet ist, mit den Schritten:
- Bereitstellen einer Sensorplattform (70), umfassend:
∘ eine Lichtquellen-Aufnahme (10), auf der eine Vielzahl von Lichtquellen-Positionen (11) vorgesehen sind, von denen jede zur Aufnahme einer chipförmigen Lichtquelle (15) ausgebildet ist, und
∘ ein Mikrolinsenarray (20), das eine Vielzahl von Mikrolinsen (21) aufweist, wobei das Mikrolinsenarray (20) und die Lichtquellen-Aufnahme (10) so zueinander angeordnet werden können, dass jede der Lichtquellen-Positionen (11) genau einer der Mikrolinsen (21) zugeordnet wird, wobei die Mikrolinsen in dem Mikrolinsenarray in dem gleichen ein- oder zweidimensionalen Raster angeordnet sind wie die Lichtquellenpositionen auf der Lichtquellenaufnahme angeordnet sind,
∘ eine Abbildungsoptik (25), und
∘ eine Detektionseinrichtung (30),
- Bilden einer Beleuchtungseinrichtung (50) durch
∘ Ausstatten der Lichtquellen-Aufnahme (10) mit chipförmigen Lichtquellen (15), wobei an mehreren der Lichtquellen-Positionen (11) der Lichtquellen-Aufnahme (10) jeweils genau eine chipförmige Lichtquelle (15) angeordnet wird und wobei die Emissionsspektren von mindestens zwei der chipförmigen Lichtquellen (15) voneinander verschieden sind, und
∘ Anordnen des Mikrolinsenarrays (20) und der mit den chipförmigen Lichtquellen (15) ausgestatteten Lichtquellen-Aufnahme (10) so zueinander, dass jeder der chipförmigen Lichtquellen (15) genau eine der Mikrolinsen (21) des Mikrolinsenarrays (20) zugeordnet wird, wobei jede der Mikrolinsen nur das Emissionslicht genau einer der chipförmigen Lichtquellen aufsammelt,
- Anordnen der Beleuchtungseinrichtung (50) und der Abbildungsoptik (25) derart, dass, beim Betreiben des Sensors (100), das von der Beleuchtungseinrichtung (50) ausgesendete Licht durch die Abbildungsoptik (25) auf ein durch den Sensor (100) zu prüfendes Wertdokument (1) abgebildet werden kann, und
- Anordnen der Detektionseinrichtung (30) derart, dass die Detektionseinrichtung (30) Detektionslicht detektieren kann, welches, beim Betreiben des Sensors (100), von dem durch die Beleuchtungseinrichtung (50) beleuchteten Wertdokument (1) ausgeht.

2. Verfahren nach Anspruch 1, **dadurch gekennzeichnet, dass**, beim Bilden der Beleuchtungseinrichtung (50), die mit den chipförmigen Lichtquellen (15) ausgestattete Lichtquellen-Aufnahme (10) und das Mikrolinsenarray (20) aneinander befestigt werden.

3. Verfahren nach einem der vorhergehenden Ansprüche, **dadurch gekennzeichnet, dass** für den Sensor (100) eine Steuerungseinrichtung (60) vorgesehen ist, die zur Ansteuerung der chipförmigen Lichtquellen (15) ausgebildet ist, und dass das Verfahren zur Herstellung des Sensors (100) außerdem das Konfigurieren der Steuerungseinrichtung (60) umfasst, um die chipförmigen Lichtquellen (15) der Beleuchtungseinrichtung (50) nacheinander ein- und auszuschalten, so dass das Wertdokument (1) nacheinander mit verschiedenen Emissionsspektren beleuchtet werden kann.

4. Verfahren nach einem der vorhergehenden Ansprüche, **dadurch gekennzeichnet, dass** aus der gleichen Sensorplattform (70) verschiedene Sensoren zur Prüfung von Wertdokumenten hergestellt werden, insbesondere mindestens ein Sensor erster Art (100A), der zur Prüfung von Wertdokumenten (1) durch Beleuchtung mit mehreren ersten Emissionsspektren ausgebildet ist, und mindestens ein Sensor zweiter Art (100B), der zur Prüfung der Wertdokumente durch Beleuchtung mit mehreren zweiten Emissionsspektren ausgebildet ist, von denen sich mindestens eines von den ersten Emissionsspektren unterscheidet.

5. Sensor (100) zur Prüfung von Wertdokumenten, insbesondere hergestellt nach dem Verfahren gemäß einem der Ansprüche 1-4, umfassend:
- eine Beleuchtungseinrichtung (50) mit
∘ mindestens zwei chipförmigen Lichtquellen (15), deren Emissionsspektren voneinander verschieden sind,
∘ einer Lichtquellen-Aufnahme (10), auf der eine Vielzahl von Lichtquellen-Positionen (11) vorgesehen sind, von denen jede zur Aufnahme einer der chipförmigen Lichtquellen (15) ausgebildet ist, wobei an mehreren der Lichtquellen-Positionen (11) jeweils genau eine der chipförmigen Lichtquellen (15) angeordnet ist, und
∘ einem Mikrolinsenarray (20), das eine Vielzahl von Mikrolinsen (21) aufweist, die in dem Mikrolinsenarray in dem gleichen ein- oder zweidimensionalen Raster angeordnet sind wie die Lichtquellenpositionen auf der Lichtquellenaufnahme angeordnet sind, wobei das Mikrolinsenarray (20) und die Lichtquellen-Aufnahme (10) derart zueinander angeordnet sind, dass jeder der auf der Lichtquellen-Aufnahme (10) angeordneten chipförmigen Lichtquellen (15) genau eine der Mikrolinsen (21) zugeordnet ist, so dass das Emissionslicht jeder der chipförmigen Lichtquellen (15) durch die der chipförmigen Lichtquelle (15) zugeordnete Mikrolinse (21) gesammelt wird, wobei jede der Mikrolinsen nur das Emissionslicht genau einer der chipförmigen Lichtquellen aufsammelt, und
- eine Abbildungsoptik (25), durch die das von der Beleuchtungseinrichtung (50) ausgesendete Licht auf ein Wertdokument (1) abgebildet werden kann, welches, beim Betreiben des Sensors (100), durch den Sensor (100) erfasst werden soll, und
- eine Detektionseinrichtung (30), die zum Detektieren von Detektionslicht ausgebildet ist, das, beim Betreiben des Sensors (100), von dem durch die Beleuchtungseinrichtung (50) beleuchteten Wertdokument (1) ausgeht.

6. Sensor (100) nach Anspruch 5, **dadurch gekennzeichnet, dass** für den Sensor (100) eine Steuerungseinrichtung (60) vorgesehen ist, die dazu eingerichtet ist, die chipförmigen Lichtquellen (15) der Beleuchtungseinrichtung (50) nacheinander ein- und auszuschalten, um das Wertdokument (1) nacheinander mit verschiedenen Emissionsspektren zu beleuchten.

7. Sensor (100) nach einem der Ansprüche 5 bis 6, **dadurch gekennzeichnet, dass** der Sensor (100) eine Vielzahl verschiedener chipförmiger Lichtquellen (15) aufweist, die, beim Betreiben des Sensors (100), nacheinander ein- und ausgeschaltet werden, um eine spektrale Intensitätsverteilung des von dem Wertdokument (1) ausgehenden Lichts zu erfassen.

8. Sensor (100) nach einem der Ansprüche 5 bis 7, **dadurch gekennzeichnet, dass** das Mikrolinsenarray (20) Befestigungsmittel (22) zum Befestigen des Mikrolinsenarrays (20) an der Lichtquellen-Aufnahme (10) aufweist.

9. Sensor (100) nach einem der Ansprüche 5 bis 8, **dadurch gekennzeichnet, dass** das Mikrolinsenarray (20) als einstückiger Körper ausgebildet ist.

10. Sensor (100) nach einem der Ansprüche 5 bis 9, **dadurch gekennzeichnet, dass** jede der Lichtquellen-Positionen (11) zur Aufnahme einer chipförmigen lichtemittierenden Diode ausgebildet ist.

11. Sensorfamilie, die mindestens einen Sensor erster Art (100A) gemäß einem der Ansprüche 5 bis 10 und mindestens einen Sensor zweiter Art (100B) gemäß einem der Ansprüche 5 bis 10 aufweist, wobei der mindestens eine Sensor erster Art (100A) zur Prüfung von Wertdokumenten durch Beleuchtung mit mehreren ersten Emissionsspektren ausgebildet ist und der mindestens eine Sensor zweiter Art (100B) zur Prüfung von Wertdokumenten durch Beleuchtung mit mehreren zweiten Emissionsspektren ausgebildet ist, von denen sich mindestens eines von den ersten Emissionsspektren unterscheidet.

12. Sensorfamilie nach Anspruch 11, **dadurch gekennzeichnet, dass** der mindestens eine Sensor erster Art (100A) und der mindestens eine Sensor zweiter Art (100B) das gleiche Mikrolinsenarray (20) und/ oder die gleiche Lichtquellen-Aufnahme (10) und/oder die gleiche Abbildungsoptik (25) und/oder die gleiche Detektionseinrichtung (30) aufweisen.

13. Sensorfamilie nach einem der Ansprüche 11 bis 12, **dadurch gekennzeichnet, dass** der mindestens eine Sensor erster Art (100A) und der mindestens eine Sensor zweiter Art (100B) aus der gleichen Sensorplattform (70), insbesondere gemäß einem der Verfahren der Ansprüche 1 bis 4, herstellbar sind, wobei zu der gleichen Sensorplattform (70) zumindest die gleiche Lichtquellen-Aufnahme (10), das gleiche Mikrolinsenarray (20), die gleiche Abbildungsoptik (25) und die gleiche Detektionseinrichtung (30) gehören.

14. Sensorfamilie nach einem der Ansprüche 11 bis 13, **dadurch gekennzeichnet, dass** die Sensoren erster Art (100A) und die Sensoren zweiter Art (100B) mit der gleichen Auswahl (S) an chipförmigen Lichtquellen (15) ausgestattet sind und dass die Sensoren zweiter Art (100B) zur Prüfung der Wertdokumente eine andere Teilmenge (B) der Lichtquellen (15) verwenden als die Sensoren erster Art (100A).

15. Sensorfamilie nach einem der Ansprüche 11 bis 13, **dadurch gekennzeichnet, dass** die Sensoren erster Art (100A) mit einer ersten Auswahl (S_{A}) an chipförmigen Lichtquellen (15A) ausgestattet sind und die Sensoren zweiter Art (100B) mit einer zweiten Auswahl (S_{B}) an chipförmigen Lichtquellen (15B) ausgestattet sind, die sich von der ersten Auswahl (S_{A}) unterscheidet.

## Claims

1. A method for manufacturing a sensor (100) which is constructed for checking value documents, having the steps of:
- making available a sensor platform (70), comprising:
∘ a light source receiving means (10) on which there are provided a multiplicity of light source positions (11), each of which is constructed for receiving a chip-shaped light source (15), and
∘ a microlens array (20) which has a multiplicity of microlenses (21), wherein the microlens array (20) and the light source receiving means (10) can be arranged relative to each other such that each of the light source positions (11) is associated with exactly one of the microlenses (21), wherein the microlenses are arranged in the microlens array in the same one- or two-dimensional grid as the light source positions are arranged on the light source receiving means,
∘ an imaging optic (25), and
∘ a detection device (30),
- forming an illumination device (50) by
∘ equipping the light source receiving means (10) with chip-shaped light sources (15), wherein at several of the light source positions (11) of the light source receiving means (10) there is respectively arranged exactly one chip-shaped light source (15), and wherein the emission spectra of at least two of the chip-shaped light sources (15) are mutually different, and
∘ arranging the microlens array (20) and the light source receiving means (10) equipped with the chip-shaped light sources (15) relative to each other such that each of the chip-shaped light sources (15) has exactly one of the microlenses (21) of the microlens array (20) associated therewith, wherein each of the microlenses collects only the emission light of exactly one of the chip-shaped light sources,
- arranging the illumination device (50) and the imaging optic (25) such that, upon operating of the sensor (100), the light emitted by the illumination device (50) can be imaged by the imaging optic (25) onto a value document (1) to be checked by the sensor (100), and
- arranging the detection device (30) such that the detection device (30) can detect detection light which, upon operating of the sensor (100), emanates from the value document (1) illuminated by the illumination device (50).

2. The method according to claim 1, **characterized in that,** upon forming of the illumination device (50), the light source receiving means (10) equipped with the chip-shaped light sources (15) and the microlens array (20) are fastened to each other.

3. The method according to any of the preceding claims, **characterized in that** there is provided for the sensor (100) a control device (60) which is constructed for activating the chip-shaped light sources (15), and that the method for manufacturing the sensor (100) moreover comprises configuring the control device (60) to successively switch the chip-shaped light sources (15) of the illumination device (50) on and off, so that the value document (1) can be illuminated successively with different emission spectra.

4. The method according to any of the preceding claims, **characterized in that** there are manufactured from the same sensor platform (70) different sensors for checking value documents, in particular at least one first-type sensor (100A) which is constructed for checking value documents (1) by illumination with several first emission spectra, and at least one second-type sensor (100B) which is constructed for checking the value documents by illumination with several second emission spectra, at least one of which differs from the first emission spectra.

5. A sensor (100) for checking value documents, in particular manufactured by the method according to any of claims 1-4, comprising:
- an illumination device (50) having
∘ at least two chip-shaped light sources (15) whose emission spectra are mutually different,
∘ a light source receiving means (10) on which there are provided a multiplicity of light source positions (11), each of which is constructed for receiving one of the chip-shaped light sources (15), wherein at several of the light source positions (11) there is respectively arranged exactly one of the chip-shaped light sources (15), and
∘ a microlens array (20) which has a multiplicity of microlenses (21) which are arranged in the microlens array in the same one- or two-dimensional grid as the light source positions are arranged on the light source receiving means, wherein the microlens array (20) and the light source receiving means (10) are arranged relative to each other such that each of the chip-shaped light sources (15) arranged on the light source receiving means (10) is associated with exactly one of the microlenses (21), so that the emission light of each of the chip-shaped light sources (15) is collected by the microlens (21) associated with the chip-shaped light source (15), wherein each of the microlenses collects only the emission light of exactly one of the chip-shaped light sources, and
- an imaging optic (25) by which the light emitted by the illumination device (50) can be imaged onto a value document (1) which, upon operating of the sensor (100), is to be captured by the sensor (100), and
- a detection device (30) which is constructed for detecting detection light which, upon operating of the sensor (100), emanates from the value document (1) illuminated by the illumination device (50).

6. The sensor (100) according to claim 5, **characterized in that** there is provided for the sensor (100) a control device (60) which is adapted to successively switch the chip-shaped light sources (15) of the illumination device (50) on and off in order to successively illuminate the value document (1) with different emission spectra.

7. The sensor (100) according to either of claims 5 to 6, **characterized in that** the sensor (100) has a multiplicity of different chip-shaped light sources (15) which, upon operating of the sensor (100), are successively switched on and off in order to capture a spectral intensity distribution of the light emanating from the value document (1).

8. The sensor (100) according to any of claims 5 to 7, **characterized in that** the microlens array (20) has fastening means (22) for fastening the microlens array (20) to the light source receiving means (10).

9. The sensor (100) according to any of claims 5 to 8, **characterized in that** the microlens array (20) is constructed as a one-pieced body.

10. The sensor (100) according to any of claims 5 to 9, **characterized in that** each of the light source positions (11) is constructed for receiving a chip-shaped light-emitting diode.

11. A sensor family which has at least one first-type sensor (100A) according to any of claims 5 to 10 and at least one second-type sensor (100B) according to any of claims 5 to 10, wherein the at least one first-type sensor (100A) is constructed for checking value documents by illumination with several first emission spectra, and the at least one second-type sensor (100B) is constructed for checking value documents by illumination with several second emission spectra, at least one of which differs from the first emission spectra.

12. The sensor family according to claim 11, **characterized in that** the at least one first-type sensor (100A) and the at least one second-type sensor (100B) have the same microlens array (20) and/or the same light source receiving means (10) and/or the same imaging optic (25) and/or the same detection device (30).

13. The sensor family according to either of claims 11 to 12, **characterized in that** the at least one first-type sensor (100A) and the at least one second-type sensor (100B) are manufacturable from the same sensor platform (70), in particular by any of the methods of claims 1 to 4, wherein the same sensor platform (70) includes at least the same light source receiving means (10), the same microlens array (20), the same imaging optic (25) and the same detection device (30).

14. The sensor family according to any of claims 11 to 13, **characterized in that** the first-type sensors (100A) and the second-type sensors (100B) are equipped with the same selection (S) of chip-shaped light sources (15), and that the second-type sensors (100B) employ a different subset (B) of the light sources (15) for checking the value documents compared to the first-type sensors (100A).

15. The sensor family according to any of claims 11 to 13, **characterized in that** the first-type sensors (100A) are equipped with a first selection (S_{A}) of chip-shaped light sources (15A) and the second-type sensors (100B) are equipped with a second selection (S_{B}) of chip-shaped light sources (15B) which differs from the first selection (S_{A}).

## Revendications

1. Procédé de fabrication d'un capteur (100) conçu pour l'examen de documents de valeur transportés, comportant les étapes :
- mise à disposition d'une plate-forme de capteurs (70), comprenant :
∘ un réceptacle de sources de lumière (10) sur lequel une pluralité de positions de sources de lumière (11) sont prévues dont chacune est conçue pour la réception d'une source de lumière (15) en forme de puce, et
∘ un groupement de microlentilles (20) comportant une pluralité de microlentilles (21), cependant que le groupement de microlentilles (20) et le réceptacle de sources de lumière (10) peuvent être agencés de telle façon l'un par rapport l'autre que chacune des positions de sources de lumière (11) est affectée à exactement une des microlentilles (21), cependant que les microlentilles dans le groupement de microlentilles sont agencées en le même réseau uni- ou bidimensionnel que les positions de sources de lumière sur le réceptacle de sources de lumière,
∘ une optique d'imagerie (25), et
∘ un équipement de détection (30),
- constitution d'un équipement d'éclairage (50) par
∘ placement de sources de lumière (15) en forme de puces dans le réceptacle de sources de lumière (10), cependant que, à plusieurs des positions de sources de lumière (11) du réceptacle de sources de lumière (10), respectivement exactement une source de lumière (15) en forme de puce est agencée et cependant que les spectres d'émission d'au moins deux des sources de lumière (15) en forme de puces sont différents l'un de l'autre, et
∘ agencement de telle façon l'un par rapport à l'autre du groupement de microlentilles (20) et du réceptacle de sources de lumière (10) doté des sources de lumière (15) en forme de puces que, à chacune des sources de lumière (15) en forme de puces, exactement une des microlentilles (21) du groupement de microlentilles (20) est affectée, cependant que chacune des microlentilles ne recueille que la lumière d'émission d'exactement une des sources de lumière en forme de puces,
- agencement de l'équipement d'éclairage (50) et de l'optique d'imagerie (25) de telle façon que, lors du fonctionnement du capteur (100), la lumière émise par l'équipement d'éclairage (50) peut être reproduite en image par l'optique d'imagerie (25) sur un document de valeur (1) à examiner par le capteur (100), et
- agencement de l'équipement de détection (30) de telle façon que l'équipement de détection (30) peut détecter de la lumière de détection qui, lors du fonctionnement du capteur (100), émane du document de valeur (1) éclairé par l'équipement d'éclairage (50).

2. Procédé selon la revendication 1, **caractérisé en ce que**, lors de la constitution de l'équipement d'éclairage (50), le réceptacle de sources de lumière (10) doté des sources de lumière (15) en forme de puces et le groupement de microlentilles (20) sont fixés l'un à l'autre.

3. Procédé selon une des revendications précédentes, **caractérisé en ce que**, pour le capteur (100), un équipement de commande (60) est prévu, lequel est conçu pour le pilotage des sources de lumière (15) en forme de puces, et que le procédé de fabrication du capteur (100) comprend en outre la configuration de l'équipement de commande (60) afin allumer et éteindre successivement les sources de lumière (15) en forme de puces de l'équipement d'éclairage (50), de telle sorte que le document de valeur (1) peut être éclairé successivement avec différents spectres d'émission.

4. Procédé selon une des revendications précédentes, **caractérisé en ce que**, depuis la même plate-forme de capteurs (70), différents capteurs destinés à l'examen de documents de valeur sont fabriqués, en particulier au moins un capteur de premier type (100A), lequel est conçu pour l'examen de documents de valeur (1) par éclairage avec plusieurs premiers spectres d'émission, et au moins un capteur de deuxième type (100B), lequel est conçu pour l'examen des documents de valeur par éclairage avec plusieurs deuxièmes spectres d'émission dont au moins un se différencie des premiers spectres d'émission.

5. Capteur (100) destiné à l'examen de documents de valeur, en particulier fabriqué suivant le procédé selon une des revendications de 1 à 4, comprenant :
- un équipement d'éclairage (50) ayant
∘ au moins deux sources de lumière (15) en forme de puces dont les spectres d'émission sont différents l'un de l'autre,
∘ un réceptacle de sources de lumière (10) sur lequel une pluralité de positions de sources de lumière (11) sont prévues dont chacune est conçue pour la réception d'une des sources de lumière (15) en forme de puces, cependant que, à plusieurs des positions de sources de lumière (11), respectivement exactement une des sources de lumière (15) en forme de puces est agencée, et
∘ un groupement de microlentilles (20) qui comporte une pluralité de microlentilles (21) qui sont agencées dans le groupement de microlentilles en le même réseau uni- ou bidimensionnel que les positions de sources de lumière sur le réceptacle de sources de lumière, cependant que le groupement de microlentilles (20) et le réceptacle de sources de lumière (10) sont agencés de telle façon l'un par rapport l'autre que, à chacune des sources de lumière (15) en forme de puces agencées sur le réceptacle de sources de lumière (10), exactement une des microlentilles (21) est affectée, de telle sorte que la lumière d'émission de chacune des sources de lumière (15) en forme de puces est recueillie par la microlentille (21) affectée à la source de lumière (15) en forme de puce, cependant que chacune des microlentilles ne recueille que la lumière d'émission d'exactement une des sources de lumière en forme de puces, et
- une optique d'imagerie (25) par laquelle la lumière émise par l'équipement d'éclairage (50) peut être reproduite en image sur un document de valeur (1), laquelle, lors du fonctionnement du capteur (100), doit être saisie par le capteur (100), et
- un équipement de détection (30) conçu pour détecter de la lumière de détection qui, lors du fonctionnement du capteur (100), émane du document de valeur (1) éclairé par l'équipement d'éclairage (50).

6. Capteur (100) selon la revendication 5, **caractérisé en ce que**, pour le capteur (100), un équipement de commande (60) est prévu, lequel est conçu pour allumer et éteindre successivement les sources de lumière (15) en forme de puces de l'équipement d'éclairage (50) afin d'éclairer successivement le document de valeur (1) avec différents spectres d'émission.

7. Capteur (100) selon une des revendications de 5 à 6, **caractérisé en ce que** le capteur (100) comporte une pluralité de différentes sources de lumière (15) en forme de puces qui, lors du fonctionnement du capteur (100), sont allumées et éteintes successivement afin de saisir une répartition spectrale d'intensité de la lumière émanant du document de valeur (1).

8. Capteur (100) selon une des revendications de 5 à 7, **caractérisé en ce que** le groupement de microlentilles (20) comporte des moyens de fixation (22) pour la fixation du groupement de microlentilles (20) au réceptacle de sources de lumière (10).

9. Capteur (100) selon une des revendications de 5 à 8, **caractérisé en ce que** le groupement de microlentilles (20) est réalisé sous forme de corps d'un seul tenant.

10. Capteur (100) selon une des revendications de 5 à 9, **caractérisé en ce que** chacune des positions de sources de lumière (11) sont conçues pour la réception d'une diode en forme de puce émettant de la lumière.

11. Famille de capteurs qui comporte au moins un capteur de premier type (100A) selon une des revendications de 5 à 10 et au moins un capteur de deuxième type (100B) selon une des revendications de 5 à 10, cependant que le au moins un capteur de premier type (100A) est conçu pour l'examen de documents de valeur par éclairage avec plusieurs premiers spectres d'émission, et le au moins un capteur de deuxième type (100B) est conçu pour l'examen des documents de valeur par éclairage avec plusieurs deuxièmes spectres d'émission dont au moins un se différencie des premiers spectres d'émission.

12. Famille de capteurs selon la revendication 11, **caractérisée en ce que** le au moins un capteur de premier type (100A) et le au moins un capteur de deuxième type (100B) comportent le même groupement de microlentilles (20) et/ou le même réceptacle de sources de lumière (10) et/ou la même optique d'imagerie (25) et/ou le même équipement de détection (30).

13. Famille de capteurs selon une des revendications 11 ou 12, **caractérisée en ce que** le au moins un capteur de premier type (100A) et le au moins un capteur de deuxième type (100B) peuvent être fabriqués depuis la même plate-forme de capteurs (70), en particulier suivant un des procédés des revendications de 1 à 4, cependant qu'appartiennent à la même plate-forme de capteurs (70) au moins le même réceptacle de sources de lumière (10), le même groupement de microlentilles (20), la même optique d'imagerie (25) et le même équipement de détection (30).

14. Famille de capteurs selon une des revendications de 11 à 13, **caractérisée en ce que** les capteurs de premier type (100A) et les capteurs de deuxième type (100B) sont pourvus de la même sélection (S) de sources de lumière (15) en forme de puces et que les capteurs de deuxième type (100B) utilisent pour l'examen des documents de valeur une autre quantité partielle (B) des sources de lumière (15) que les capteurs de premier type (100A).

15. Famille de capteurs selon une des revendications de 11 à 13, **caractérisée en ce que** les capteurs de premier type (100A) sont pourvus d'une première sélection (S_{A}) de sources de lumière (15A) en forme de puces et que les capteurs de deuxième type (100B) sont pourvus d'une deuxième sélection (S_{B}) de sources de lumière (15B) en forme de puces qui se différencient de la première sélection (S_{A}).
